# EUROPEAN PATENT APPLICATION

(11) **EP 4 744 650 A1**
(43) Date of publication of application: **20.05.2026**
(21) Application number: 24840109.3
(22) Date of filing: 11.07.2024
(51) Int. Cl.: A61K 9/16, A61K 31/495, A61P 25/18

(54) **METHOD FOR PREPARING CARIPRAZINE MICROSPHERES, MICROSPHERES PREPARED THEREBY, AND PHARMACEUTICAL COMPOSITION COMPRISING MICROSPHERES**

(30) Priority: 12.07.2023 KR 20230090238
(71) Applicant: Aulbio Co., Ltd., Seongnam-si, Gyeonggi-do 13105 (KR)
(72) Inventor: KIM, Cherng Ju, Beaumont, California 92223 (US); AN, Tae Kun, Yongin-si, Gyeonggi-do 16990 (KR); KIM, A Ram, Seoul 05667 (KR); KIM, Seo A, Suwon-si, Gyeonggi-do 16509 (KR); YEON, Je Won, Suwon-si, Gyeonggi-do 16675 (KR)
(74) Representative: dompatent
(86) International application number: PCT/KR2024/009943
(87) International publication number: WO 2025/014299

(57) **Abstract**

The present invention provides a method for preparing cariprazine microspheres, comprising the steps of: (a) preparing a dispersed phase by dissolving or dispersing cariprazine or a pharmaceutically acceptable salt thereof and at least one biocompatible polymer in a mixed solvent of methanol and methylene chloride, (b) adding the prepared dispersed phase into a continuous phase and stirring to form microspheres, and (c) removing the solvent; microspheres prepared by the method; and a pharmaceutical composition comprising the microspheres.

## Description

### [Technical Field]

This application claims the benefit of priority to Korean Patent Application No. 10-2023-0090238 filed on July 12, 2023, the entire contents of which are incorporated herein as part of this specification.

The present invention relates to a method for preparing cariprazine microspheres, microspheres prepared thereby, and a pharmaceutical composition comprising the microspheres.

### [Background Art]

Cariprazine is a dopamine D3-preferring D3/D2 receptor partial agonist. Cariprazine is used for the treatment and/or prevention of pathological diseases that require modulation of dopamine receptors, such as psychoses (e.g., schizophrenia, schizoaffective disorder, etc.), abuse of drugs (e.g., alcohol, cocaine, nicotine, opioids, etc.), cognitive impairment accompanying schizophrenia (including positive symptoms such as delusions and hallucinations; and negative symptoms such as lack of momentum and social withdrawal, and cognitive symptoms such as problems with posture and memory), mild cognitive deficits, moderate cognitive deficits, dementia, psychotic states associated with dementia, eating disorders (e.g., bulimia nervosa, etc.), attention deficit disorder, hyperactivity disorder in children, psychotic depression, mania, paranoia, delusional disorder, dyskinesia (e.g., Parkinson's disease, neuroleptic-induced parkinsonism, tardive dyskinesia) anxiety, sexual dysfunction, sleep disorders, vomiting, aggression, autism, etc.

Cariprazine is typically delivered via immediate-release oral formulations. However, because cariprazine is a drug that requires long-term administration, improved administration convenience is needed for immediate-release oral formulations. Therefore, research is being conducted on extended-release formulations, nanoparticle formulations, or delivery systems of cariprazine microspheres that can provide extended drug release, ease of administration, and high patient compliance.

For example, Korean Laid-open Patent Publication No. 10-2022-0127254 proposes a long-term injection formulation and delivery system for cariprazine. However, the disclosed technology only proposes a formulation capable of drug release for 10 days, limiting its ability to provide extended drug release, ease of administration, and high patient compliance.

### [Prior Art Document]

### [Patent Document]

Korean Laid-open Patent Publication No. 10-2022-0127254.

### [Disclosure]

### [Technical Problem]

The inventors of the present invention have conducted repeated research to solve the above-mentioned problems of the prior art and have developed microspheres in which cariprazine is encapsulated at a high concentration and continuously released for a long period of time without an initial burst.

Therefore, the present invention aims to provide microspheres that exhibit stable drug release for a long period of time and can maintain an effective concentration of cariprazine in the blood for a certain period of time, thereby extending the drug administration cycle, increasing patient compliance with medication, and reducing side effects caused by rapid initial drug release, as well as a method for preparing the same and a pharmaceutical composition comprising the same.

### [Technical Solution]

In order to solve the above problem, the present invention provides a method for preparing microspheres, comprising the steps of:
(a) preparing a dispersed phase by dissolving or dispersing cariprazine or a pharmaceutically acceptable salt thereof and at least one biocompatible polymer in a mixed solvent of methanol and methylene chloride;
(b) adding the prepared dispersed phase to a continuous phase and stirring to form microspheres; and
(c) removing the solvent.

In addition, the present invention
provides microspheres prepared by the above method, comprising cariprazine or a pharmaceutically acceptable salt thereof, and a biocompatible polymer.

In addition, the present invention
provides a pharmaceutical composition comprising the microspheres of the present invention.

### [Advantageous Effects]

The microspheres of the present invention exhibit stable drug release over a long period of time, allowing cariprazine to be maintained at an effective concentration in the blood for a certain period of time, thereby extending the drug administration cycle and enhancing patient compliance with medication. Furthermore, the initial burst is minimized, thereby alleviating side effects caused by rapid initial drug release.

In addition, the method for preparing microspheres of the present invention provides the effect of efficiently preparing microspheres having excellent loading capacity and encapsulation rate, and minimized initial burst and sustained release properties.

In addition, the pharmaceutical composition of the present invention provides an excellent effect in the prevention or treatment of schizophrenia, mania, bipolar disorder and the likeby including the microspheres.

### [Description of Drawings]

FIG. 1 is a scanning electron microscope (SEM) photograph of the morphology of microspheres prepared in Example 1 of the present invention, and
FIG. 2 is a graph illustrating the results of measuring the size of microspheres prepared in Example 1 of the present invention.
FIG. 3 is a graph illustrating the results of an in vitro release test of microspheres prepared in Example 1 of the present invention, and
FIG. 4 is a graph illustrating the results of an in vitro release test of microspheres prepared in Comparative Example 1 of the present invention.

### [Best Mode]

Hereinafter, the present invention will be described in more detail.

All technical terms used in the present invention, unless otherwise defined, are used in the same meaning as commonly understood by those skilled in the art in the relevant fields of the present invention. Furthermore, methods or samples similar or equivalent to those described herein are also included within the scope of the present invention. The contents of all publications cited herein are incorporated herein by reference in their entirety.

In the present invention, cariprazine or a pharmaceutically acceptable salt thereof may be collectively referred to as cariprazine.

The present invention relates to a method for preparing microspheres, comprising the steps of:
(a) preparing a dispersed phase by dissolving or dispersing cariprazine or a pharmaceutically acceptable salt thereof and one or more biocompatible polymers in a mixed solvent of methanol and methylene chloride;
(b) adding the prepared dispersed phase to a continuous phase and stirring to form microspheres; and
(c) removing the solvent.

The cariprazine is a compound whose chemical name is trans-N-{4-[2-[4-(2,3-dichlorophenyl)-piperazin-1-yl]-ethyl]-cyclohexyl}-N',N'-dimethylurea.

The cariprazine is a dopamine D3-preferring D3/D2 receptor partial agonist. Cariprazine can be used for the treatment and/or prevention of pathological diseases that require modulation of dopamine receptors, such as psychoses (e.g., schizophrenia, schizoaffective disorder, etc.), abuse of drugs (e.g., alcohol, cocaine, nicotine, opioids, etc.), cognitive impairment accompanying schizophrenia (including positive symptoms such as delusions and hallucinations; and negative symptoms such as lack of momentum and social withdrawal, and cognitive symptoms such as problems with posture and memory), mild cognitive deficits, moderate cognitive deficits, dementia, psychotic states associated with dementia, eating disorders (e.g., bulimia nervosa, etc.), attention deficit disorder, hyperactivity disorder in children, psychotic depression, mania, paranoia, delusional disorder, dyskinesia (e.g., Parkinson's disease, neuroleptic-induced parkinsonism, tardive dyskinesia), anxiety, sexual dysfunction, sleep disorders, vomiting, aggression, autism, etc.

In one embodiment of the present invention, the pharmaceutically acceptable salt of the cariprazine may be selected from the group consisting of, for example, hydrochloride, sulfate, phosphate, methane sulfonate, camphor sulfonate, oxalate, maleate, succinate, citrate, formate, hydrobromide, benzoate, tartrate, fumarate, salicylate, mandelate, and carbonate of cariprazine, but is not limited thereto.

The cariprazine salts can be prepared by conventional techniques, for example, by adding an acid to free cariprazine to convert it into an acid addition salt, or by converting one acid addition salt into another salt.

In one embodiment of the present invention, in the step (a), the mixed solvent of methanol and methylene chloride may be used in a mixing weight ratio of 1:1 to 8.5, 1:1.1 to 7, 1:1.1 to 6, or 1:5 to 6. If the weight ratio of methylene chloride is less than 1, a problem may arise in which a severe pore structure is generated in the microspheres or the microspheres are not properly formed, and if it exceeds 8.5, a problem may arise in which an initial burst occurs, which is not preferable.

In one embodiment of the present invention, the ratio of the combined weight of cariprazine or a pharmaceutically acceptable salt thereof and the biocompatible polymer to the weight of the mixed solvent in the step (a) may be 1:1 to 10, 1:1 to 8, 1:1 to 5, or 1:1 to 3. If the weight ratio of the mixed solvent is less than 1, the drug may precipitate and, when forming microspheres, the drug may be distributed outside the microspheres, resulting in a problem of high initial burst release. If it exceeds 10, the microspheres may not be formed well or a problem of initial burst may occur, which is not preferable.

In one embodiment of the present invention, in the step (a), the biocompatible polymer may be included in an amount of 10 to 50 wt% or 15 to 45 wt% based on the total weight of the dispersed phase. If the weight ratio of the biocompatible polymer is less than 10 wt%, problems such as the drug not being encapsulated in the microspheres or the microspheres not being formed properly may occur, and if it exceeds 50 wt%, the viscosity of the dispersed phase may be too high, which may cause problems such as the microspheres not being formed, which is not preferable.

In the present invention, microspheres mean microspheres prepared using a biocompatible polymer in which the cariprazine or a pharmaceutically acceptable salt thereof is encapsulated, and are simply referred to as cariprazine-containing microspheres, cariprazine microspheres, or microspheres. As long as cariprazine or a pharmaceutically acceptable salt thereof is encapsulated in microspheres prepared using a biocompatible polymer, they are all included in the scope of the present invention, regardless of the type of biocompatible polymer used.

In the present invention, the "biocompatible polymer" refers to a polymer whose safety is ensured, as the polymer and its degradation products do not cause cytotoxicity or inflammatory reactions in the body, and is also referred to simply as a polymer in this specification.

In the present invention, the biocompatible polymer can be selected based on its intrinsic viscosity. A suitable intrinsic viscosity may be 0.1 to 1.9 dL/g, preferably 0.1 to 1.2 dL/g, and more preferably 0.1 to 1.0 dL/g. When the intrinsic viscosity is less than 0.1 dL/g, the biocompatible polymer may decompose too quickly, making it difficult to sustain the release of cariprazine for a desired period of time. When the intrinsic viscosity exceeds 1.9 dL/g, the polymer may decompose too slowly, making the amount of cariprazine released too small.

In one embodiment of the present invention, the biocompatible polymer may be at least one selected from polylactic acid, polylactide, polylactic-co-glycolic acid, polylactide-co-glycolide (PLGA), polyphosphazine, polyiminocarbonate, polyphosphoester, polyanhydride, polyorthoester, copolymers of lactic acid and caprolactone, polycaprolactone, polyhydroxyvalerate, polyhydroxybutyrate, polyamino acids, and copolymers of lactic acid and amino acids.

Among these, polylactic acid is particularly preferred.

In one embodiment of the present invention, the weight of cariprazine or a pharmaceutically acceptable salt thereof encapsulated in the microspheres obtained according to the preparation method may be 70 wt%, 80 wt%, 85 wt%, or 90 wt% or more relative to the weight of cariprazine or a pharmaceutically acceptable salt thereof dissolved in the step (a). In this case, the upper limit may be 100 wt% or less, 98 wt% or less, 96 wt% or less, or 94 wt% or less.

The step (b) is a step of solidifying microspheres by dispersing the dispersed phase prepared in the step (a) in an external continuous phase to prepare an emulsion solution (O/W).

In the step (b), a hydrophilic polymer may be included as a surfactant, and the type thereof is not particularly limited, and any one that can help the dispersed phase including cariprazine or a pharmaceutically acceptable salt thereof and a biocompatible polymer to form a stable droplet dispersed phase within the external continuous phase may be used.

The hydrophilic polymer may preferably be selected from the group consisting of methylcellulose, polyvinylpyrrolidone, carboxymethylcellulose, lecithin, gelatin, polyvinyl alcohol, polyoxyethylene-polyoxypropylene block copolymer, polyoxyethylene sorbitan fatty acid ester, polyoxyethylene castor oil derivative, and mixtures thereof, and polyvinyl alcohol may be preferably used.

In the step (b), the external continuous phase may be a hydrophilic polymer aqueous solution of 0.1 to 5% (w/v), preferably 0.1 to 3% (w/v), wherein the weight average molecular weight of the hydrophilic polymer may be 7,000 to 40,000, and the degree of hydrolysis may be 80 to 90%.

In the step (b), the dispersed phase containing cariprazine or a pharmaceutically acceptable salt thereof and a biocompatible polymer prepared in the step (a) is added to the external continuous phase containing the hydrophilic polymer by drop-by-drop or by using an in-line mixer, and stirred vigorously to prepare an emulsion solution (O/W). In this process, the cariprazine or a pharmaceutically acceptable salt thereof is encapsulated into biocompatible polymer microspheres.

Next, the solvent is removed in the step (c), and the desired microspheres can be obtained after conventional filtration and washing. That is, if necessary, a step of washing the obtained microspheres with an organic solvent such as ethanol may be further included to enhance the initial release inhibition effect.

The present invention also
provides microspheres prepared by the above method, comprising cariprazine or a pharmaceutically acceptable salt thereof, and a biocompatible polymer.

With regard to the microspheres, all of the contents described in the above microsphere preparation method can be applied. Therefore, any duplicate contents may be omitted below.

In one embodiment of the present invention, the biocompatible polymer may be included in an amount of 70 to 99 wt%, 72 to 90 wt%, or 73 to 80 wt% based on the total weight of the microspheres. When the weight ratio of the biocompatible polymer is less than 72 wt%, the distribution of cariprazine or a pharmaceutically acceptable salt thereof may relatively increase, which may cause problems such as initial excessive release or inability to maintain the medicinal efficacy for a desired period of time, and when it exceeds 99 wt%, the dissolution amount of cariprazine may not be effectively achieved, and the amount to be administered to a patient may become too large, making administration itself difficult or impossible.

In one embodiment of the present invention, the cariprazine or a pharmaceutically acceptable salt thereof may be included in an amount of 1 to 30 wt%, 5 to 30 wt%, or 20 to 30 wt% based on the total weight of the microspheres.

In one embodiment of the present invention, the microspheres may be capable of sustaining the release of cariprazine for 10 days or more, 14 days or more, 21 days or more, or 28 days or more. Conventional cariprazine microspheres, however, have a drawback in that they do not provide convenient administration to patients because the release of cariprazine does not last for more than 10 days. The microspheres of the present invention can extend the duration of medicinal efficacy to at least one month, thereby significantly improving the convenience of administration.

In one embodiment of the present invention, the cariprazine contained in the microspheres may be released in an amount of 10 wt% or less, preferably 7 wt% or less, and more preferably 6 wt% or less, within one day. In addition, the release amount within 5 days may be 20 wt% or less, preferably 18 wt% or less, and more preferably 15 wt% or less. In addition, the release amount within 10 days may be 30 wt% or less, preferably 28 wt% or less, and more preferably 25 wt% or less.

In one embodiment of the present invention, the microspheres may contain impurities (components other than cariprazine or a pharmaceutically acceptable salt thereof, and the biocompatible polymer) contained during the preparing process in an amount of 5 wt% or less based on the total weight of the microspheres. Taking such impurities into account, the content of the biocompatible polymer may be reduced by the content of the impurities.

In one embodiment of the present invention, the microspheres can provide the encapsulated cariprazine or a pharmaceutically acceptable salt thereof in a controlled or extended release form. The controlled or extended release form may be understood to have the same meaning as "sustained release," "controlled release," or "delayed release."

The present invention also
provides a pharmaceutical composition comprising the microspheres and a pharmaceutically acceptable carrier.

The pharmaceutical composition may be used for the treatment and/or prevention of pathological diseases requiring modulation of dopamine receptors, such as psychosis (e.g., schizophrenia, schizoaffective disorder, etc.), abuse of drugs (e.g., alcohol, cocaine, nicotine, opioids, etc.), cognitive impairment accompanying schizophrenia (including positive symptoms such as delusions and hallucinations; and negative symptoms such as lack of momentum and social withdrawal, and cognitive symptoms such as problems with posture and memory), mild cognitive deficits, moderate cognitive deficits, dementia, psychotic states associated with dementia, eating disorders (e.g., bulimia nervosa, etc.), attention deficit disorder, hyperactivity disorder in children, psychotic depression, mania, paranoia, delusional disorder, dyskinesia (e.g., Parkinson's disease, neuroleptic-induced parkinsonism, tardive dyskinesia) anxiety, sexual dysfunction, sleep disorders, vomiting, aggression, autism, etc.

In one embodiment of the present invention, the pharmaceutical composition may be formulated for parenteral administration.

The pharmaceutical composition for parenteral administration may contain the microspheres alone or may further comprise a pharmaceutically acceptable carrier for parenteral administration, which may be conventionally added to the pharmaceutical composition. Furthermore, it may further contain an excipient or diluent. The carriers include all types of solvents, dispersion media, oil-in-water or water-in-oil emulsions, aqueous compositions, liposomes, microbeads, and microsomes.

The carrier for parenteral administration may include water, suitable oils, saline solution, aqueous glucose and glycol, etc., and may further include stabilizers and preservatives.

Suitable stabilizers may include antioxidants such as sodium bisulfite, sodium sulfite, or ascorbic acid. Suitable preservatives may include benzalkonium chloride, methyl- or propyl-paraben, and chlorobutanol.

The pharmaceutical composition of the present invention may further include, in addition to the above components, lubricants, wetting agents, sweeteners, flavoring agents, emulsifiers, suspending agents, etc. Other pharmaceutically acceptable carriers and preparations may be referred to as described in the following literature (Remington's Pharmaceutical Sciences, 19th ed., Mack Publishing Company, Easton, PA, 1995).

The parenteral administration method of the present invention may be administered to a patient (e.g., a person in need of such a drug) or other animals by intravenous, intramuscular, intraarterial, intramedullary, intrathecal, intracardiac, transdermal, subcutaneous, intraperitoneal, intranasal, enteral, intravaginal, intrapulmonary, suppository, topical, sublingual or rectal administration, but is not limited thereto.

In the present invention, 'treatment' means any act of improving or beneficially altering an (acute or chronic) disease, disorder, and symptoms resulting therefrom by administering a pharmaceutical composition. In addition, the 'treatment' broadly includes the meaning of 'prevention,' whereby 'prevention' means any act of suppressing or delaying the onset of a disease and symptoms resulting therefrom by administering a pharmaceutical preparation. The 'treatment' includes, for example, the obstruction, alleviation, improvement, cessation, suppression, delay, and reversal of the progression of an (acute or chronic) disease, disorder, and symptoms resulting therefrom.

The dosage of the microspheres or pharmaceutical composition of the present invention will vary depending on factors such as the frequency and duration of administration, the nature and severity of the disease to be treated, the age and general health of the subject (host), and the subject's (host's) tolerance to the active ingredient. Considering these factors, those skilled in the art will be able to determine an appropriate effective dosage of the composition of the present invention. The pharmaceutical composition of the present invention is not particularly limited in its formulation, route of administration, or method of administration, as long as it exhibits the effects of the present invention.

Hereinafter, the present invention will be described in detail using examples. However, the examples according to the present invention may be modified in various ways, and the scope of the present invention should not be construed as being limited to the examples described below. These examples of the present invention are provided to more fully explain the present invention to those of ordinary skill in the art.

### Example 1: Preparation of cariprazine microspheres

0.3 g of cariprazine (manufacturer: Synaptics labs) and 0.9 g of biocompatible polymer (Resormer 203H, manufacturer: Evonik) were weighed and placed in a vial, and 0.475 g of methanol (manufacturer: Deoksan) and 2.394 g of methylene chloride (manufacturer: Deoksan) were mixed to complete the dispersed phase.

The continuous phase used a 0.5% polyvinyl alcohol aqueous solution (manufacturer: Sigma-Aldrich, molecular weight: 9,000-10,000). 1,000 ml of the continuous phase was placed in a production tank, and the dispersed phase prepared above was injected at 25°C, and microspheres were prepared using a homogenizer (Kinematica, Switzerland).

Afterwards, an organic solvent was removed by heating the mixture at 25°C for 5 hours and then increasing the temperature to 35°C for 19 hours. The prepared microspheres were washed several times with water for injection, and the residual polyvinyl alcohol was removed, and the microspheres were freeze-dried.

### Comparative Example 1: Preparation of cariprazine microspheres

0.3 g of cariprazine (manufacturer: Synaptics Labs) and 0.9 g of a biocompatible polymer (Resormer 203H, manufacturer: Evonik) were weighed and placed in a vial, and mixed with 26.6 g of methylene chloride (manufacturer: Deoksan) to form the dispersed phase.

The continuous phase used a 0.5% of polyvinyl alcohol aqueous solution (manufacturer: Sigma-Aldrich, molecular weight: 9,000-10,000). 1,000 ml of the continuous phase was placed in a production tank, and the dispersed phase prepared above was injected at 25°C. Microspheres were prepared using a homogenizer (Kinematica, Switzerland).

Afterwards, the organic solvent was removed by heating the mixture at 25°C for 5 hours and then increasing the temperature to 35°C for 19 hours. The prepared microspheres were washed several times with water for injection, and the residual polyvinyl alcohol was removed, and the microspheres were freeze-dried.

### Test Example 1: Morphology confirmation of cariprazine microspheres

To confirm the morphology of the microspheres prepared in Example 1 and Comparative Example 1, it was analyzed using a scanning electron microscope (SEM). Approximately 20 mg of microspheres were fixed to an aluminum stub and mounted in a SEM (equipment name: Hitachi TM4000 Plus) to observe the surfaces of the microspheres. All images were observed with a 5 KeV electron beam at a magnification of approximately 500X.

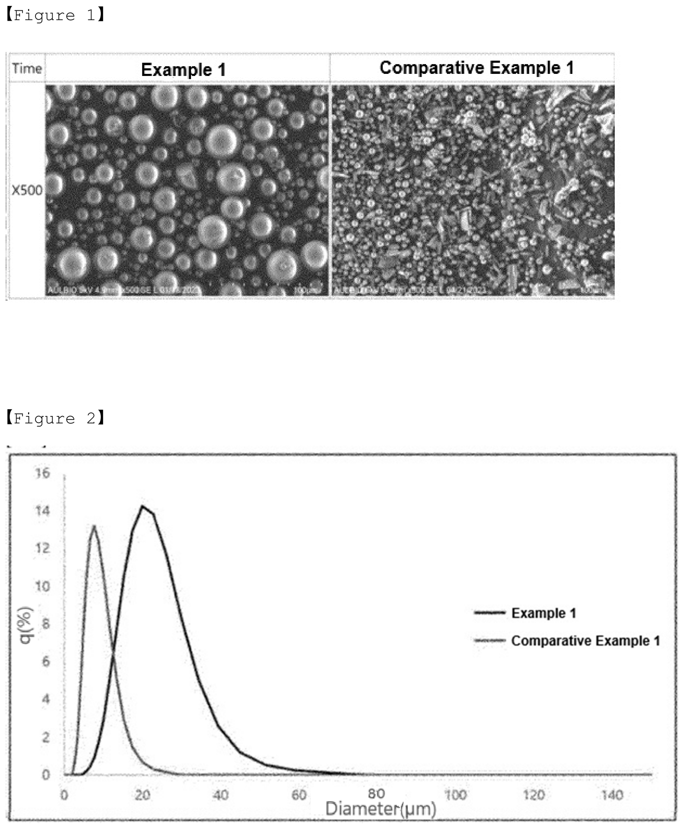

The above observation results are shown in FIG. 1. As can be seen in FIG. 1, in the case of Example 1, it can be seen that no drug crystals were precipitated outside the spherical microspheres due to the use of a small amount of solvent. On the other hand, in the case of Comparative Example 1, it can be seen that a large amount of crystalline drug was precipitated outside the microspheres.

### Test Example 2: Size measurement of cariprazine microspheres

The sizes of the microspheres prepared in Example 1 and Comparative Example 1 were measured using a particle size analyzer (PSA). Approximately 100 mg of each microsphere was placed in a 1.5 mL EP tube, 1 mL of deionized water was added, and vortexing was performed for 1 minute. The microspheres were then placed in a PSA (equipment name: Horiba LA-960S2) cuvette, and the sizes of the microspheres were measured. The measurement results are shown in Table 1 below and FIG. 2.

**[Table 1]**

| | D10 | D50 | D90 | Span |
|---|---|---|---|---|
| Example 1 | 11 *µ*m | 19 *µ*m | 30 *µ*m | 0.99 *µ*m |
| Comparative Example 1 | 4 *µ*m | 7 *µ*m | 12 *µ*m | 1.09 *µ*m |

From the results in Table 1 and FIG. 2, it can be confirmed that the microspheres of Example 1 are easier to prepare into microspheres of a target size by using a co-solvent to reduce the total amount of solvent compared to when using only methylene chloride, and that drug precipitation does not occur, making it easier to control release. Therefore, Example 1 of the present invention is superior to the microspheres of Comparative Example 1 in this respect.

### Test Example 3: Confirmation of loading and encapsulation rates of cariprazine microspheres

Approximately 12 mg of each of the microspheres prepared in Example 1 and Comparative Example 1 were placed in a 20 mL flask, completely dissolved in 10 mL of acetonitrile, and then filtered through a 0.22 µm RC syringe filter until the volume was adjusted to the mark with distilled water. The solution was detected by a UV-visible spectrophotometer using HPLC (equipment name: Agilent 1260). The column packing was performed with L1, internal diameter of 4.6 mm x 250 mm, and thickness of 3.5 µm.

The test results are shown in Table 2 below.

**[Table 2]**

| | Cariprazine Loading rate (%) | Cariprazine Encapsulation rate(%) |
|---|---|---|
| Example 1 | 22.69 | 90.74 |
| Comparative Example 1 | 22.62 | 90.48 |

### Test Example 4: In vitro release test and initial release rate evaluation

Approximately 1 mg of each of the microspheres prepared in Example 1 and Comparative Example 1 was placed in a vial, and 8 mL of pH 6.0 PBS solution was added. The vial was stirred at 100 rpm and maintained at 37°C. To measure the release amount over a certain period of time, 8 mL of the supernatant was filtered through a 0.22 µm RC filter and used as a test solution, and 8 mL of the new release solution was placed in the vial. The test solution was detected by a UV visible spectrophotometer using HPLC (equipment name: Agilent 1260). The column packing was performed with L1, an internal diameter of 4.6 mm x 250 mm, and a thickness of 5 µm.

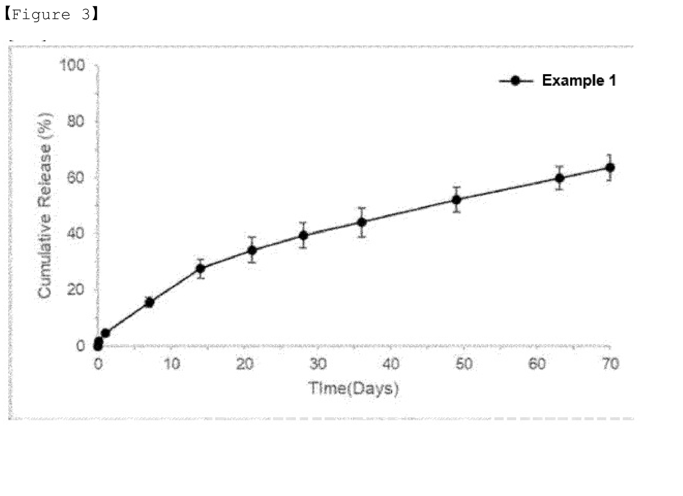

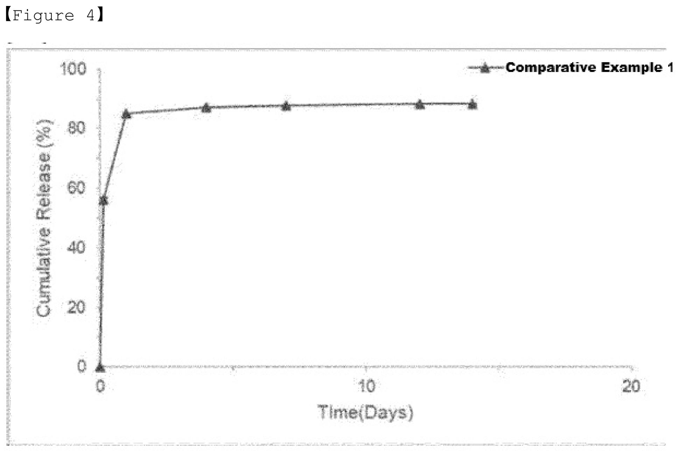

The test results are shown in Table 3 below and FIGS. 3 and 4.

**[Table 3]**

| | Initial release rate (%) (3 hours) | Initial release rate (%) (1 day) |
|---|---|---|
| Example 1 | 2% | 5% |
| Comparative Example 1 | 56% | 85% |

From FIG. 3, it can be seen that the microspheres prepared in Example 1 steadily released 28% of cariprazine for 14 days without an initial burst. Conversely, from FIG. 4, it can be seen that the microspheres prepared in Comparative Example 1 rapidly released 56% of cariprazine over the first 6 hours of the test.

Therefore, from these results, it can be confirmed that the cariprazine microspheres of Example 1 of the present invention may utilize a small amount of solvent by using a methanol and methylene chloride co-solvent, thereby preventing drug precipitation and significantly reducing the initial burst. Furthermore, it can be seen that they exhibit excellent sustained-release properties by continuously releasing the drug for 14 days.

## Claims

1. A method for preparing microspheres, comprising the steps of:
(a) preparing a dispersed phase by dissolving or dispersing cariprazine or a pharmaceutically acceptable salt thereof and one or more biocompatible polymers in a mixed solvent of methanol and methylene chloride;
(b) adding the prepared dispersed phase to a continuous phase and stirring to form microspheres; and
(c) removing the solvent.

2. The method for preparing microspheres of claim 1, wherein the mixed solvent of methanol and methylene chloride in the step (a) has a mixed weight ratio of 1:1 to 8.5.

3. The method for preparing microspheres of claim 1, wherein the ratio of the combined weight of cariprazine or a pharmaceutically acceptable salt thereof and a biocompatible polymer to the weight of the mixed solvent in the step (a) is 1:1 to 10.

4. The method for preparing microspheres of claim 1, wherein the biocompatible polymer is contained in an amount of 10 to 50 wt% based on the total weight of the dispersed phase in the step (a).

5. The method for preparing microspheres of claim 1, wherein the biocompatible polymer is at least one selected from polylactic acid, polylactide, polylactic-co-glycolic acid, polylactide-co-glycolide (PLGA), polyphosphazine, polyiminocarbonate, polyphosphoester, polyanhydride, polyorthoester, copolymer of lactic acid and caprolactone, polycaprolactone, polyhydroxyvalerate, polyhydroxybutyrate, polyamino acid, and copolymer of lactic acid and amino acid.

6. The method for preparing microspheres of claim 2, wherein the biocompatible polymer is polylactic acid.

7. The method for preparing microspheres of claim 1, wherein the weight of cariprazine or a pharmaceutically acceptable salt thereof encapsulated in the microspheres obtained according to the manufacturing method is 70 wt% or more to the weight of cariprazine or a pharmaceutically acceptable salt thereof dissolved in the step (a), based on the cariprazine free base.

8. Microspheres prepared by the method of claim 1, comprising cariprazine or a pharmaceutically acceptable salt thereof, and a biocompatible polymer.

9. The microspheres of claim 8, wherein the biocompatible polymer is contained in an amount of 70 to 99 wt% based on the total weight of the microspheres.

10. The microspheres of claim 8, wherein the biocompatible polymer is at least one selected from polylactic acid, polylactide, polylactic-co-glycolic acid, polylactide-co-glycolide (PLGA), polyphosphazine, polyiminocarbonate, polyphosphoester, polyanhydride, polyorthoester, copolymer of lactic acid and caprolactone, polycaprolactone, polyhydroxyvalerate, polyhydroxybutyrate, polyamino acid, and copolymer of lactic acid and amino acid.

11. The microspheres of claim 8, wherein the biocompatible polymer is polylactic acid.

12. The microspheres of claim 8, wherein the microspheres are microspheres in which the release of cariprazine continues for more than 10 days.

13. The microspheres of claim 8, wherein the cariprazine contained in the microspheres has a release amount of 10 wt% or less within 1 day.

14. A pharmaceutical composition comprising the microspheres of claim 8 and a pharmaceutically acceptable carrier.

15. The pharmaceutical composition of claim 14, wherein the pharmaceutical composition is for the prevention or treatment of schizophrenia, mania, and bipolar disorder.
